# EUROPEAN PATENT APPLICATION

(11) **EP 0 630 632 A2**
(43) Date of publication of application: **28.12.1994**
(21) Application number: 94304544.3
(22) Date of filing: 22.06.1994
(51) Int. Cl.: A61F 13/15

(54) **Disposable undergarment**

(30) Priority: 22.06.1993 JP 33610/93
(71) Applicant: UNI-CHARM CORPORATION, Kawanoe-shi Ehime-ken (JP)
(72) Inventor: Fujioka, Yoshihisa, Mitoyo-gun, Kagawa-ken (JP); Ono, Yoshio, Kawanoe-shi, Ehime-ken (JP); Yamaki, Rumi, Kawanoe-shi, Ehime-ken (JP); Kaji, Miwako, Kawanoe-shi, Ehime-ken (JP)
(74) Representative: Warren, Anthony Robert

(57) **Abstract**

An undergarment according to the invention comprises basic cloth (1) having an elastic stretchability,at least in width , and an absorbent pad (10) having an elastic stretchability, at least in width, and bonded to the inner surface of the basic cloth. During use, the basic cloth (1) adapts itself to movement of the wearer and the absorbent pad (10) elastically adapts to elastic movements of the basic cloth, thereby avoiding collapse of the absorbent pad.

## Description

The present invention relates to a disposable undergarment of the pants type formed by bonding front and rear bodies along respective opposite side edges at a waist level such as incontinence diapers, baby diapers, baby training pants and sanitary pants.

Such an undergarment of the prior art usually employs elastically stretchable nonwoven fabric at least partially as top- and backsheets and a liquid-absorbent core mainly consisting of fluffy pulp to be sandwiched between the top- and backsheets.

However, the elastic stretchability of said top- and backsheets as a whole is limited by the liquid-absorbent core because said liquid-absorbent core is substantially non-stretchable and the liquid-absorbent core is sometimes collapsed or partially broken due to the elastic behavior of the top- and backsheets. The collapse of the liquid-absorbent core will give the wearer an uncomfortable feeling or will significantly reduce its absorptivity for liquid excretion.

It is an object of the invention to eliminate the above-mentioned problems by providing the liquid-absorbent core with an elastic stretchability.

The object set forth above is achieved, according to the invention, by a disposable undergarment comprising a basic cloth made of fibrous nonwoven fabric elastically stretchable at least in length and width and provided with a waist-opening and a pair of leg-openings, wherein said undergarment further comprises an absorbent pad .having an elastic stretchability at least in width and intermittently bonded to the inner surface of said undergarment.

Preferably, said absorbent pad comprises top- and backsheets both having an elastic stretchability at least in width and a liquid-absorbent core having an elastic stretchability at least in width, said core being sandwiched between said top- and backsheets and made at least of synthetic fibre having an elastic stretchability.

Preferably, said absorbent pad comprises top- and backsheets both having an elastic stretchability at least in width and an integrally molded liquid-absorbent core which is substantially non-stretchable and sandwiched between said top- and backsheets so that said top- and backsheets may be elastically stretchable at least in width with respect to said core.

The basic cloth elastically adapts itself to movement of the wearer during use and the absorbent pad also elastically adapts to such elastic movement of the basic cloth.

The invention will be described by way of example with reference to the accompanying drawings, in which:
Fig. 1 is a front view showing an embodiment of an undergarment constructed according to the invention;
Fig. 2 is a developed view showing, partially broken away, the inside of the undergarment shown by Fig. 1; and
Fig. 3 is an enlarged scale sectional view taken along a line X-X in Fig. 2.

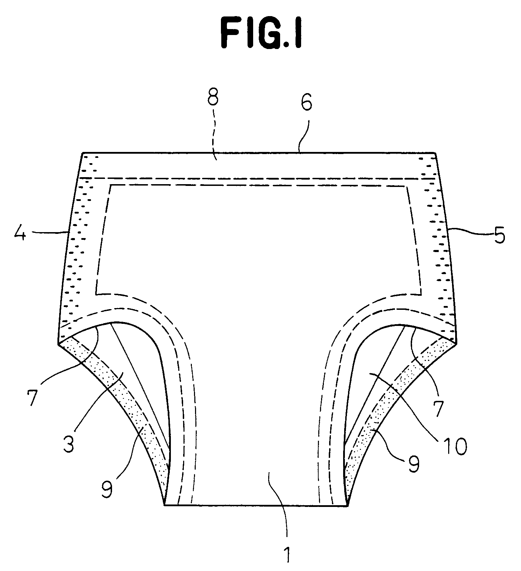

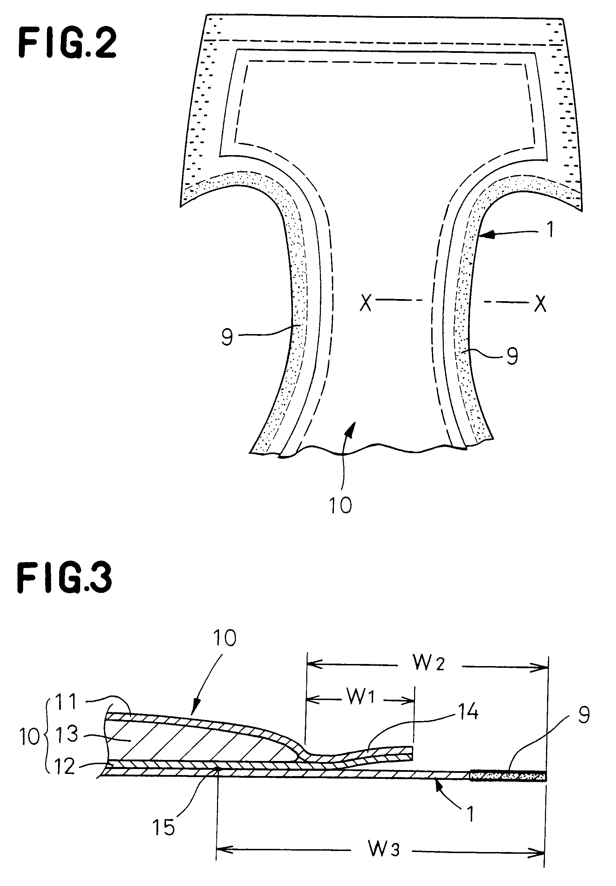

Referring to Figs. 1 through 3, there is shown an embodiment of the invention. An undergarment is cut out from fibrous nonwoven fabric used as a basic cloth 1 having an elastic stretchability both in length and width (preferably, an elastic stretchability higher in width than in length) and has a waist-opening 6 and a pair of leg-openings 7 formed by bonding opposite side edges 4, 5 of a front body 2 to corresponding opposite side edges 4, 5 of a rear body 3 in a dotted pattern.

The basic cloth 1 of the front and rear bodies 2, 3 may be formed, for example. from a web of heat crimped synthetic fibre having its component fibres being entangled or intertwined by subjecting them to fluid jet or intermittently bonding together under pressure and heat.

The waist-opening 6 is provided on its inner periperal surface with an elastic member 8 in the form of a tape so as to be elastically stretchable in the circumferential direction. The elastic member 8 is preferably of a color different from that of the basic cloth 1 for the front and rear bodies 2, 3 because the elastic member 8 will be seen through said basic cloth and thereby an aesthetic effect will be obtained.

Synthetic resin of hot melt type presenting rubber-like elasticity in its cured state, which has been conventionally employed for bonding members of a disposable diaper, is applied to the inner peripheral surface of each leg-opening 7 fully to the outer peripheral edges thereof to form a ribbon-like elastic zone 9 having a stretching stress of 50 to 130 g. As a result, a region of the leg-opening 7 occupied by the ribbon-like elastic zone 9 has a stretching stress higher than that in the remaining region of the basic cloth 1. Obviously, it is possible to make such comparative measurement of the stretching stress on the same-sized pieces of the respective regions cut off from the basic cloth 1.

It is also possible to use, instead of said resin, so-called rubber elastomer which is elastic material in the form of liquid or gel presenting a rubber-like elasticity in its cured state or after heat treatment.

To coat each leg-opening 7 fully to its outer peripheral edge with the fluidized elastic material, such as the above-mentioned resin or rubber elastomer, the basic cloth 1 is coated, before the region to form each leg-opening 7 is cut off from the basic cloth 1, with said fluidized elastic material so that the ribbon-like elastic zone 9 may be defined over a surplus coated area,and a line along which said region is to be cut may be located within said surplus coated area.

If it is apprehended that the ribbon-like elastic zone 9 formed on the inner peripheral surface of the leg-opening 7 might give the wearer's skin an uncomfortable feeling, at least the zone 9 may be covered with a ribbon-like nonwoven fabric of elastic fibre, or said zone 9 may be provided on the outer peripheral surface of the leg-opening 7.

The elastic material such as the previously mentioned resin used to form the ribbon-like elastic zone 9 may be selected from those having colors other than that of the basic cloth 1 for the front and rear bodies 2, 3,to provide the leg-opening 7 with a trimming pattern and thereby to obtain an aesthetic effect just as in the case of the waist-opening 6.

An absorbent pad 10 is laid upon the inner surface of the basic cloth 1. The absorbent pad 10 has a contour substantially similar to that of the basic cloth 1 and a size smaller than that of the basic cloth 1. The absorbent pad 10 comprises liquid-permeable top- and backsheets 11, 12,both made of fibrous nonwoven fabric having an elastic stretchability at least in width,and a liquid-absorbent core 13 sandwiched therebetween having a contour smaller than that of these sheets,and an elastic stretchability at least in width so that the absorbent pad 10 may be elastically stretched as a whole at least in width. It should be understood that he elastic stretchability of the absorbent pad 10, particularly of the liquid-absorbent core 13, may be lower than that of the basic cloth 1 to such a degree that the liquid-absorbent core 13 can be free from an undesirable deformation such as collapse or breakage due to a stretch behavior of the basic cloth 1. Regions of the top- and backsheets extending outward from outer peripheral edges of the core 13 are bonded together by means of adhesive or suitable welding means to form side flaps 14 each having a width W₁ on opposite sides of the absorbent pad 10. The backsheet 12 may be formed, if desired, by a liquid-impermeable sheet such as water-proofed fibrous nonwoven fabric or plastic film.

The liquid-absorbent core 13 comprises a mixture of thermoplastic crimped fibre, hydrophilic fibre such as fluffy pulp or rayon, and high absorption polymer powder, in which component fibres have been partially welded together to form a netty structure. The mixture is then molded under heating into a mat-like core 13 substantially collapse-free during normal use which is, in turn, bonded to the top- and backsheets 11, 12 by means of adhesive or suitable welding means preferably so as to obtain an integral assembly. The thermoplastic crimped fibre is preferably of 15 to 40% by weight. With the thermoplastic crimped fibre lower than 15% by weight, a desired elastic stretchability will not be obtained and, with the thermoplastic crimped fibre higher than 40% by weight, a content of liquid-absorbent material such as fluffy pulp will be inconveniently reduced, resulting in a disadvantageously deficient absorptivity.

Alternatively, the absorbent pad 10 may be realized by molding, under heating, a mixture of thermoplastic fibre, hydrophilic fibre such as fluffy pulp or rayon, and high absorption polymer powder into a mat-like core 13 which is substantially collapse-free during normal use and substantially non-stretchable, then bonding the topsheet 11 but not the backsheet 12 to the core 13 so that the core 13 is sandwiched between these sheets and only the backsheet 12 is stretchable together with the basic cloth 1.

The core 13 is shaped to have a thickness gradually decreasing from a region adjacent its outer peripheral edges to its outer peripheral edges so that an abrupt difference in level which otherwise would appear between the basic cloth 1 and the outer peripheral edges of the absorbent pad 10 may be avoided and, in consequence, a zone adjacent each leg-opening 7 may fit well around the wearer's leg. Such desired shape of the core 13 can be achieved, for example, by appropriate allotment of core materials. The absorbent pad 10 is intermittently (preferably in dotted pattern) bonded to the basic cloth 1 by means of adhesive or suitable welding means (not shown). A distance (width) W₂ from the inner side edge of each side flap 14 to the corresponding outer side edge of the basic cloth 1 depends on the rigidity of the absorbent pad 10 and is preferably 10mm or larger to maintain a desired contractile force of the ribbon-like elastic zone 9. For the same reason and to maintain an area of the basic cloth that can be elastically stretched independently of the absorbent pad 10 as large as possible, it is preferred that each outermost side edge 15 of the region over which the absorbent pad 10 is bonded to the basic cloth 1 is located inside the inner side edge of the corresponding side flap 14, and a distance W₃ from each outermost side edge 15 to the corresponding outer side edge of the basic cloth 1 is 15 mm or larger.

With the undergarment constructed as described hereinabove according to the invention, the basic cloth elastically adapts itself to the movement of the wearer during use and the absorbent pad also elastically adapts itself to the expansion and contraction of said basic cloth, so the absorbent pad is free from collapse as well as partial breakage due to the stretch behavior of said basic cloth which might give the wearer an uncomfortable feeling and significantly reduce its absorptivity for liquid excretion.

## Claims

1. A disposable undergarment comprising a basic cloth made of fibrous nonwoven fabric elastically stretchable at least in length and width and provided with a waist-opening and a pair of leg-openings, said undergarment further comprising an absorbent pad having an elastic stretchability at least in width and intermittently bonded to the inner surface of said undergarment.

2. A disposable undergarment according to Claim 1, wherein said absorbent pad comprises top- and backsheets both having an elastic stretchability at least in width and a liquid-absorbent core having an elastic stretchability at least in width and sandwiched between said sheets; and wherein said core is made at least of synthetic fibre having elastic stretchability.

3. A disposable undergarment according to Claim 1, wherein said absorbent pad comprises top- and backsheets both having an elastic stretchability at least in width and an integrally molded liquid-absorbent core which is substantially non-stretchable and sandwiched between said top- and backsheets so that said top- and backsheets may be elastically stretched at least in width with respect to said core.
